# EUROPEAN PATENT SPECIFICATION

(11) **EP 1 887 997 B1**
(45) Date of publication and mention of the grant of the patent: **08.06.2011**
(21) Application number: 06726702.1
(22) Date of filing: 12.04.2006
(51) Int. Cl.: A61F 5/441, A61F 5/445

(54) **COLOSTOMY ARRANGEMENTS**
KOLOSTOMIEANORDNUNGEN
ENSEMBLES DE COLOSTOMIE

(30) Priority: 14.04.2005 GB 0507501; 24.05.2005 GB 0510597; 19.12.2005 GB 0525718
(43) Date of publication of application: 20.02.2008
(73) Proprietor: BMTLIFESTYLE LIMITED, Four Oakes Sutton, Coldfield West Midlands B75 5BY (GB)
(72) Inventor: PLAYDON, Michael Albert, Walsall WS7 2BJ (GB)
(74) Representative: Parnham, Kevin
(86) International application number: PCT/GB2006/001301
(87) International publication number: WO 2006/109034

(56) References cited:
- EP-A- 0 868 892
- EP-A- 0 985 390
- WO-A1-01/00260
- WO-A1-97/34646
- US-A- 4 203 445
- US-A- 6 015 399

## Description

The present invention relates to colostomy arrangements and more particularly with regard to evacuation of colostomy arrangements through stoma exits.

In order to treat numerous medical complaints it is necessary to perform colostomy surgical procedures in order to remove a part of the intestine and/or bowel. As a result of such colostomy procedures an individual is left with a section of intestine which is directly evacuated through a stoma exit in the abdomen wall. In such circumstances individuals can continue relatively normal lives and faeces and other waste pass through the stoma into a receptacle bag attached to the stoma.

It will be understood that natural bowel movements are facilitated and helped by gravitational factors. However, as a stoma exit is generally located as indicated above in an abdomen wall, gravity has less effect. In any event, an individual after colostomy surgery must relearn how to control and regulate their bowl movements. Previously, such control has been achieved principally through trial and error. It will be understood that in situations where wastes will be collected in bags it is desirable to achieve regularity with regard to bowel movements. Such regularity with bowel movement is normally advantageous but with respect to individuals after colostomy surgery, the ability to predictably expect to change their bag has clear benefits. It will also be understood that as part of the natural digestion process gases will be created and individuals and colostomy arrangements must be able to cope with such gas effusions.

It is known to provide colostomy bags which incorporate vent valves and assemblies. As described in documents European patent number 0868892, US patent number 4203445 and US patent number 6015399 gas vents which may be operated through press button release or plug mechanisms are known. In such circumstances rather than a simple cover to a filter a valve is provided which extends over the filter and the plug removed or the pressure build up released through a push button mechanism. Such an approach does not allow bowel movement training and depends upon uncomfortable build up of gas pressure which ideally should be avoided. Stimulating gas flow for co-effusion with solid wastes will allow a patient to more rapidly acquire necessary understanding capabilities with regard to managing their condition,

In accordance with aspects of the present invention there is provided a colostomy arrangement as described in claim 1.

The invention with its gaseous withdrawal pump is used to stimulate bowel movements in a colostomy patient.

The gaseous withdrawal pump may comprise a funnel arranged to be secured to the adaptor. The pump can be operated to draw gas through the stoma for a desired period of time to remove gas by effusion from accumulated bowel waste in a bowel and such effusion stimulated by co withdrawal movements of the bowel.

The gaseous withdrawal pump may comprise an impeller operable to draw gas with the pump.

Typically, the hood substantially surrounds the whole arrangement with an aperture through which the stoma opening projects.

Typically, the bag is wet proof. Possibly, the bag is formed from a plastics material or a rubber material.

Typically, the hood is fibrous. Possibly, the hood is formed from a textile or non woven felt sheet.

Possibly, the adaptor is sealable. Possibly, the adaptor is sealable by an adhesive patch extending across the adaptor. Alternatively, the adaptor is sealable by a flap displaceable when gaseous withdrawal is provided. Typically, the adaptor comprises a single hole. Alternatively, the adaptor comprises a plurality of holes or perforations.

Generally, the filter is located directly opposite the stoma opening.

Advantageously, the filter is directly aligned with the adaptor.

Generally, the filter is formed from a fibrous material. Generally, the fibrous material is provided by an air laid paper or non woven fabric or wool or foam.

Possibly, the filter is impregnated with a scenting agent.

Advantageously, the filter is integrally secured to the bag. Potentially, the filter provides a moisture barrier when wet.

Possibly, the filter and/or the adaptor are arranged to regulate rates of gaseous withdrawal in use.

Typically, the desired period of time is sufficient to draw gas for bowel waste de-gasification.

Generally, the bag arrangement is retained attached to the stoma when the withdrawal pump is removed.

There can be selective use of the withdrawal pump in order to train bowel movements and control.

Aspects of the present invention will now be described by way of example only with reference to the accompanying drawings in which:-
Fig. 1 is a schematic side view of a bag arrangement in accordance with a first embodiment of certain aspects of the present invention;
Fig. 2 is a schematic view of a stoma opening side of the bag depicted in Fig. 1;
Fig. 3 is a schematic plan view of an adaptor side of the bag arrangement depicted in Figs. 1 and 2;
Fig. 4 is a schematic cross section of a bag arrangement as depicted in Figs. 1 to 3 in use;
Fig. 5 is a schematic illustration of a method and process by which bowel movement training and control is facilitated;
Fig. 6 is a schematic side view of an alternative bag arrangement;
Fig. 7 is a side perspective view of the bag arrangement depicted in Fig. 6 in use;

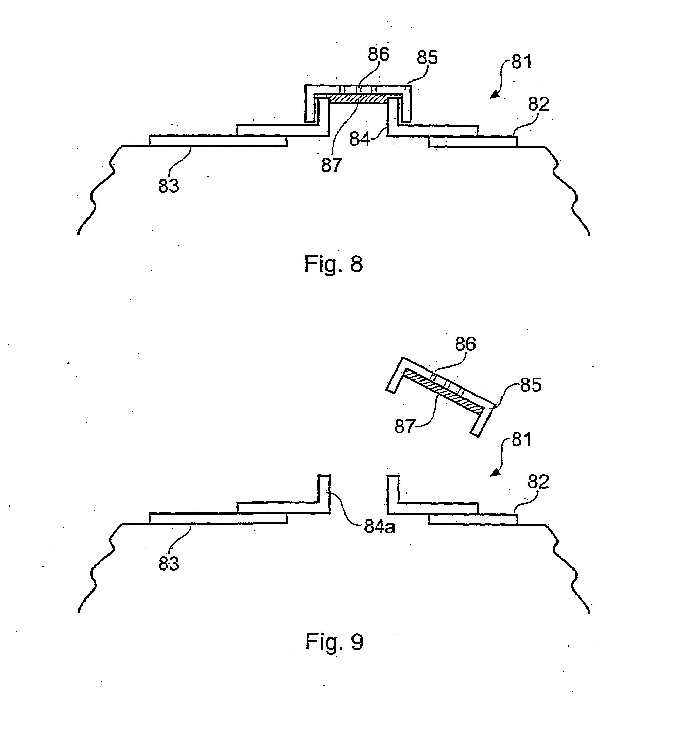

Fig. 8 is a schematic cross section of a stoma closure; and,
Fig. 9 is a schematic cross-section of the arrangement depicted in Fig. 8 with a cap removed to allow evacuation of faeces

Convenient use of bags by association with a stoma exit after colostomy surgery is important in order to maintain patient dignity and to allow those patients to continue with as normal a lifestyle as possible. Such convenience involves allowing predictability with respect to bowel movements and when such bowel movements occur allowing convenient accommodation of such movements.

It will be understood that typically in a user gravity plays a significant part in stimulating bowel movements but such influence may be more limited once colostomy surgery has been performed. In such circumstances, regaining understanding and predictability with regard to bowel movement is important to an individual who has had colostomy surgery. Waste within an intestine will generally comprise agglomerations of waste digestion solids as well as gases and liquids. Gases can present problems as will be described later which must be accommodated but also can be utilised in order to stimulate bowel movements. It will be understood that the gases and potentially some liquids are more mobile than agglomerated solids. In such circumstances by stimulating gas movement and removal from the bowel wastes that this may cause some breakdown to co draw the solids, liquids and gas towards a stoma exit and through a stoma opening in the bag.

It will be understood as a result of colostomy surgery generally a stoma exit opening will be formed to extend through the abdomen of a patient. This stoma opening provides an outlet for wastes. Typically, the stoma exit is a plastic or other element surgically secured to the intestine and projecting in the order of 10 to 15mm beyond the abdomen to provide a conduit through which wastes can be excised. Clearly, these wastes must be contained and generally a stoma bag is secured over the stoma to receive the wastes. Natural digestive reflexes and peristaltic motions may force the wastes into the bag. Nevertheless, particularly during the early stages of bowel movement training, it is difficult for a patient to achieve control of their bowel movements and therefore accidents and embarrassment can occur. One potential source of such irregularity is gas build up within the remainder intestine system introducing unpredictability with respect to bag expansion requirements and flow rates.

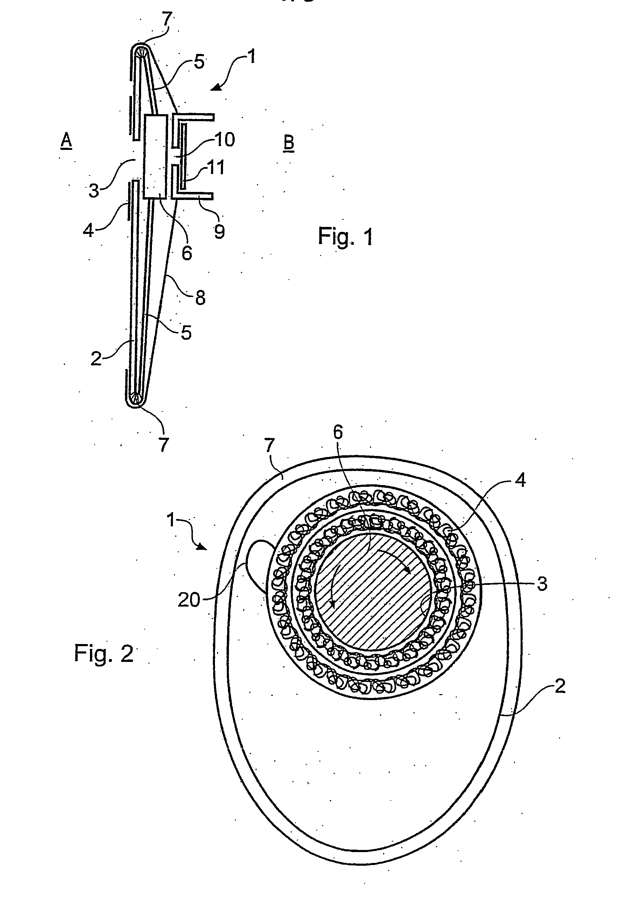

Certain aspects of the present invention provide a bag arrangement which can be used in a method and process for bowel movement control. Fig. 1 provides a schematic side view of a bag arrangement 1 in accordance with certain aspects of the present invention. The arrangement 1 comprises a bag which is generally wet proof and therefore formed from a plastics or other material. This bag will receive the intestinal waste for subsequent disposal. The bag comprises a face sheet 2 in which a stoma opening 3 is provided to allow the arrangement 1 to be secured to a stoma exit. Normally, adhesive patches 4 are provided to ensure that the arrangement 1 is adhered to the abdomen of a user and so retain association between the opening 3 and a stoma (not shown). The bag is formed with a backer layer 5 which in accordance with certain aspects of the present invention includes a vent filter 6.

The arrangement 1 also includes a hood or cover 8 which at least extends over the backer layer 5 incorporating the vent filter 6. It will be appreciated that this hood 8 may also extend around the ends and be associated with the welds 7 in order to provide a cover for the bag comprising layers 2, 5 in order to provide user comfort and discretion. The hood 8 is normally formed from a fibrous material which may take the form of textile or a non woven sheet for user comfort and touch. Within the hood 8 an adaptor 9 is provided. As will be described later this adaptor 9 allows a gas evacuation pump to be secured to the arrangement 1 in order to draw gas through the filter 6 and a stoma opening 3 in order to stimulate and control bowel movements.

As depicted in Fig. 1 generally the stoma opening 3, the filter 6 and the adaptor 9 with an opening 10 are all advantageously aligned with each other to create a gas draw path through the opening 3, filter 6 and adaptor opening 10.

It will be understood that providing the arrangement in a relatively sterile state is important and so typically a patch 11 will be securable over the opening 10.

The vent filter 6 generally allows gas to be drawn through it but presents a barrier to solid wastes and typically expands to limit liquid transmission across the filter 6. It will be understood that the arrangement 1 will be regularly replaced and therefore generally the arrangement will be replaced before any substantial liquid has passed across the filter 6.

The filter 6 is generally formed from a fibrous material of a suitable sanitary grade such as absorbent materials. The filter is generally formed from an air laid paper or non woven fabric or wool. As the flow is generally one way it is will be understood that the filter 6 could also incorporate an absorbent powder to further limit the possibility of liquid transfer across the filter in use. Even more advantageously the filter 6 may incorporate scenting agents in order to apply a masking scent to any gaseous flow across the filter 6 and therefore avoid potentially embarrassing odours.

Normally the filter 6 will be integrally secured with the backer layer 5 to make sure that the barrier does not rupture in use.

As will be described later gentle natural gas drawing through the adaptor 9, filter 6 and opening 3 must be achieved. In such circumstances the filter 6 and/or the adaptor 9 may be arranged to ensure that overly aggressive gas extraction does not occur. For example, the patch 11 may be arranged to allow the number of holes in the adaptor 9 to be varied. Furthermore, as indicated above generally the hood or cover 8 is formed of a fibrous or textile material and therefore may allow for some air ingress to limit the suction capacity and therefore aggressiveness of the drawing gas flow through the filter 6.

Fig. 2 provides a schematic view from the direction of side A of the arrangement depicted in Fig. 1. Thus, as can be seen a stoma opening 3 provides an aperture to which a stoma exit can be secured. The filter 6 can be seen through the opening 3 and the patch 4 located around the opening 3 can be seen with its storage cover secured. This storage cover will be removed by pulling a tag 20 in order to expose the adhesive layer of the patch 4 to allow it to be secured to a user's abdomen. In such circumstances, it will be noted that the aperture 3 and the patch 4 will generally be located to one side of the arrangement 1 such that in use wastes can drop through the opening into a sump portion of the bag for collection.

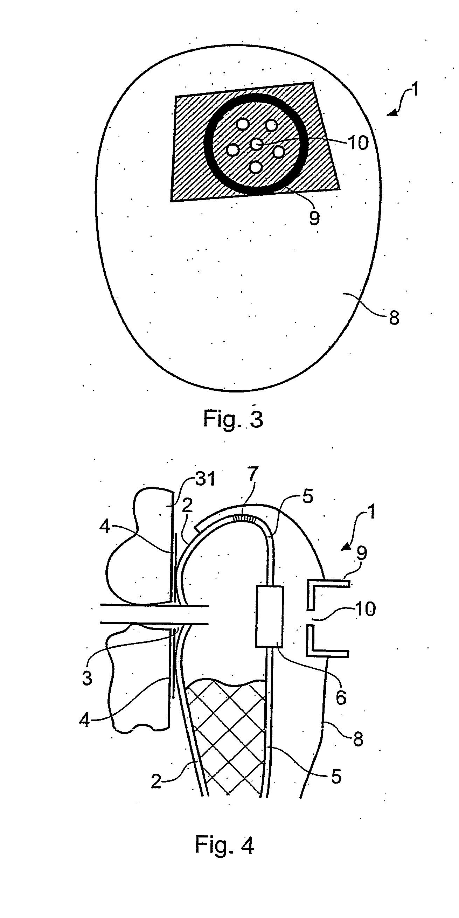

Fig. 3 illustrates the arrangement 1 depicted in Figs. 2 and 3 from a side B depicted in Fig. 1. Thus, the arrangement 1 has a hood or cover 8 in which the adapter 9 is secured, again to one side of the arrangement 1 for consistency with the filter 6 and opening 3 (not shown). The adapter 3 in such circumstances in use will be presented outwardly from a user's abdomen and allow a gas evacuation pump to be secured to the adapter in use.

As indicated above, the adapter can comprise a variety of small perforations or holes in a pepper-pot formation to allow gas effusion through the filter 6. Alternatively, as depicted in Fig. 1 a single central hole can be provided depending upon operational requirements.

As indicated above, generally the adapter 9 and, in particular, its opening 10, filter 6 and stoma opening 3, will be aligned. However, where desirable, these components may be misaligned within the arrangement in order to provide an indirect gas flow path which may facilitate regulation of gas flow to avoid overly aggressive effusion of natural gas.

Fig. 4 provides a schematic cross-section of the bag arrangement 1 depicted in Figs. 1 to 3 in use. Thus, the arrangement is secured on a stoma exit 30 projecting from a user's abdomen 31. Waste 32 has been forced or drawn through the stoma 30 into the bag comprising layers 2, 5. The filter 6 in the back of layer 5 has prevented liquid and solid wastes 32 escaping from the bag created by the layers 2, 5. The adapter 9 remains associated with the hood or cover 8. In such circumstances, the aperture 10, if allowed to remain open, will allow any gas passing through the filter 6 to escape. Alternatively, as described previously, a patch 11 may be secured across the opening 10 to prevent release of gas. Alternatively, this patch may be rendered relatively porous to allow slow release of gas build-up between the hood or cover 8 and the back of layer 5. In any event, as described above, generally the hood or cover 8 will be formed from a fibrous material and, therefore, it will be understood that this material will be porous and allow slow release of gas passing through the filter 6 and dwelling in the space between the back of 5 and the hood or cover 8. In order to improve acceptability, the fibrous material from which the hood or cover 8 is formed or a patch 11 secured across the aperture 10 may incorporate scenting agents to mask the smell of the gas.

The present invention is used for stimulating bowel movements and draining with regard to such bowel movements. In such circumstances, the bag arrangement 1 as described above with regard to Figs. 1 to 4 can be utilised. A funnel-like member can be applied over a stoma exit in order to draw gaseous flow through the stoma exit from an attached remaining intestine. This stimulation will act to co-draw the solid/liquid wastes by effusion of gas from these wastes towards the gas evacuation mechanism. This mechanism comprises a simple impeller air or gas pump with a relatively gentle gas drawing rate. Such pumps have been utilised previously for drawing ear wax through horn elements from human ears.

It will be appreciated with a base funnel and associated gas evacuation pump no consideration is made with regard to inadvertent spluttering of waste into the pump which might create an unsightly mess. In such circumstances a bag arrangement 1 as described in Figs. 1 to 4 will be advantageous in providing a process by which natural gas evacuation can occur through the stoma exit whilst avoiding a spluttering of wastes.

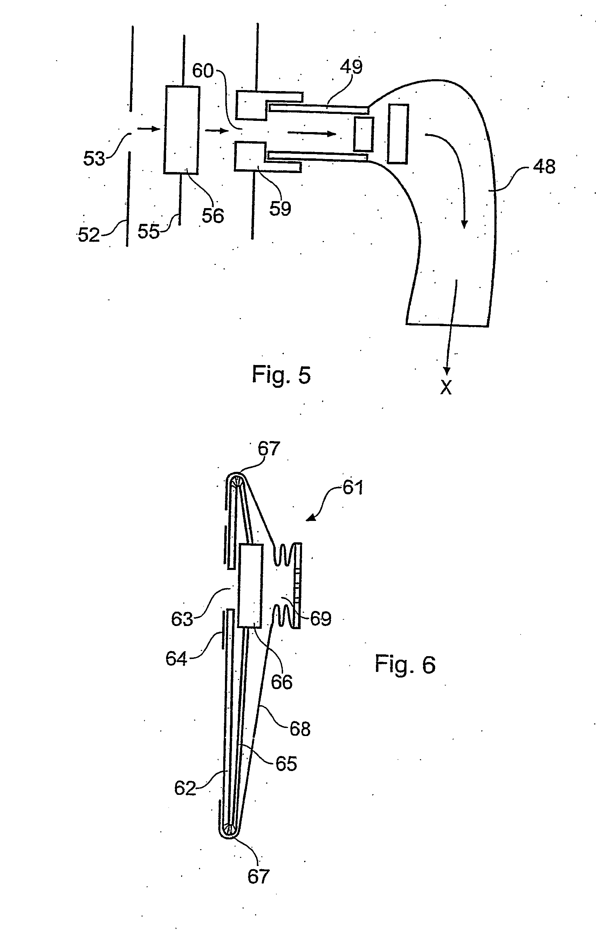

Fig. 5 provides a schematic illustration of a method and process by which bowel movements can be stimulated and individual training with regard to achieving periodicity with respect to bowel movements. As indicated above, natural gases within the intestine system are a variable factor which can create problems with respect to stoma bags. In such circumstances, by evacuating natural gases within the remaindered intestine and bowel allow stimulation of bowel movements and waste flow. In Fig. 5, a filter 56 is positioned as previously in a backer layer 55 of a bag formed with a layer 52 incorporating a stoma opening aperture. A hood or cover 58 includes an adapter 59 having an aperture 60 in order to draw air and natural gas in the direction of arrowhead X through the filter 56, aperture 60 and into a funnel 49 associated with a gas withdrawal pump 48. The pump 48 includes an impeller driven by an electric motor in order to draw air in the direction of the arrowheads X along with natural gases through the filter 56. As described previously, generally the hood or cover 58 will be fibrous and therefore allow some external air leakage into the flow path as well as drawing natural gas through the filter 56 such that there is a gentle evacuation of gas. Typically, the funnel 49 is secured upon a mounting for the pump 48 which will include a wire mesh or other relatively open screen to prevent any fibrous or debris which could become detached entering the pump 48.

In use, as described previously, a bag arrangement will be secured upon a stoma through the stoma opening 53 and any closure patch or otherwise removed across the adaptor 59 in order to expose the aperture 60. The funnel 49 will then be secured to the adaptor 59 and the pump 48 switched on. Operation of the pump will generally be for a pre-determined time period which is normally in the order of five minutes. This time period may vary dependent upon requirements so that during a timing period as indicated more stimulation of bowel movement 2 gaseous withdrawal will be provided whilst if simple removal of actual gas flatulence is required operation of the pump may only occur for a short period of time. In either event, as described previously, drawing the natural gas from the waste will cause typically co-movement and, therefore, stimulate bowel movement. In any event, as will be appreciated, trapped wind can cause pain and, therefore, removal using the present invention will be advantageous.

Once the pre-determined time period for gas evacuation has been performed, it will be understood that the pump 48 will be detached and typically a closure path or flap located across the aperture 60. However, natural gas pressure may still allow transmission of some gas across the filter 56 and this will be gradually released through the aperture 60 and potentially the fibrous open-pore nature of the hood or cover 48. It will be understood that the pump 48 may have an integral funnel 49 or other means of coupling to the adapter 59 including a bespoke male/female coupling or simple attachment nozzle.

It will be understood that the natural gases evacuated through the filter 56 may have an undesirable smell and in such circumstances the filter 56 may incorporate a scenting agent which will be picked up by the gas flow across the filter 56 in order to create a more pleasant odour. Furthermore, the hood or cover 58 may also incorporate a scent which may be actively picked up by the gas or provide a simple background pleasant odour.

It will be understood that generally the gas evacuated may have a humidity or moisture content. In such circumstances this moisture will tend to be collected by the filter 56 in order to act as a barrier to prevent such moisture being released. The filter 56 will, therefore, progressively become increasingly wetted. In such circumstances the filter 56 may incorporate absorbent powders or other means including absorbent fibres in order to reduce the moisture content in the gas evacuated through the aperture 60 into the pump 48. These fibres may also have anti-microbial and disinfectant actions. It will also be understood in view of the moisture content variation in the filter 56 it may be possible to provide an identifier when the filter 56 has become saturated or indication as to when it will be advisable to stop gas evacuation. This indication may take the form of a colour change in the filter 56 or potentially other parts of the bag arrangement. This colour change could be achieved through an absorbent agent which changes its colour between dry and wet states.

As indicated above, once the pump is removed, the bag stays in place secured to the stoma through the aperture 53 and as indicated previously typically an adhesive patch secured to the user's abdomen. In such circumstances, the bag formed by the layers 52, 55 can act as previously as a receptacle for waste passed through the stoma and collected normally. The filter 56 particularly when wet will act as a barrier to moisture and solids movement through the bag and, therefore, ensure containment of the waste. As indicated above, the filter 56 may be scented to further reduce any noxious odours. The filter 56 is generally integrally formed with the layer 55 to limit any potential for rupture at the junction between the filter 56 and the layer 55 forming the receptacle bag for wastes. Once the bag is full, it is removed in the normal way and disposed of appropriately.

It will be understood that stoma waste collecting bags associated with stomas can be relatively intrusive and, therefore, during normal daytime may be unacceptable. Collection bags are typically used overnight or at other convenient times whilst less intrusive bags are used for daytime periods. These daytime bags will be of a smaller nature and typically will be designed to accept one to three hours' waste bowel movements. In such circumstances the bags have a lower capacity but it will be understood that the voluminous nature of gas effluxions can create problems. In such circumstances it has been known to provide a small sponge-type element within bags which is designed to actively absorb the natural gas emissions. Furthermore, it has been known previously to provide a filter and cover which incorporate pin-hole apertures to allow slow release of natural gas. In accordance with some aspects of the present invention, a bag arrangement for colostomy stomas is provided which can more readily accommodate gas emissions.

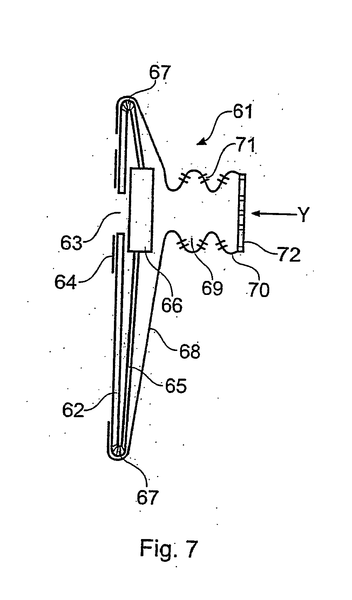

Figs. 6 and 7 respectively show a schematic cross-section a bag arrangement 61 in its initial state in Fig 6 and in use in Fig. 7.

The bag arrangement 61 comprises a contact layer 62 incorporating a stoma aperture 63 and adhesive flap 64 to allow the bag arrangement 61 to be secured to a user's stoma. A backer layer 65 incorporates a filter vent 66 which as illustrated is most conveniently directly aligned with the stoma aperture 63. A receptacle bag is created through edge-welding 67 so that wastes passing through the aperture 63 are collected in the bag between the layers 62, 65. The filter 66 allows gas to pass through into a capture area formed by a cover 68. Again this cover 68 will generally be secured about the periphery of the arrangement 61 so that gas is contained. It will be understood that the gas will preferentially pass through the filter 66 but in situations where there is limited solid/liquid waste expansion of the bag will principally be achieved by the gas. Release of the gas into the collection area between the back of the backer layer 65 and the cover 68 will again be retained until released.

The cover 68 incorporates a concertina pocket 69 to act as the capture area for the natural gas passing through the filter 66. Having a concertina form, it will be understood that moulded sides or walls 70 unfold to increase the captive capacity of the pocket 69 to allow the natural gas to be collected. In accordance with certain aspects of the present invention, apertures or perforations are provided in the concertina pocket so that when the concertina pocket is opened an external pressure force in the direction of arrowhead Y can be applied in order to force natural gas out of the pocket 69.

In the above circumstances, if the bag arrangement 61 is full of liquid/solid waste then depression in the direction of arrowhead Y will not reduce the size of the arrangement 61 and a user will, therefore, be aware that it is necessary to change their bag 61. Alternatively, if depression in the direction of arrowhead Y results in a collapse of the arrangement 61, as the natural gas is forced through the apertures 71 it will be known that expansion was due to that natural gas and, therefore, the bag can still be used for a period of time.

In order to reduce odours, the filter 66 as well as the material from which the cover 70 is formed may incorporate a scenting agent.

Normally, the concertina fold walls 70 will be relatively flexible to enable ease of opening to increase the size of the pocket 69. A top 72 to the pocket 69 will generally be more robust to enable adequate depression pressure Y to be applied. Apertures can be provided in the top 72 but most conveniently the apertures will be in the side walls 70 so that the apertures open for normal aspirate release of gas as well as to allow unobstructed depression by depression force Y such as user's fingers for release of natural gas.

In use the arrangement 61 as previously will comprise placing the arrangement 61 on a stoma exit through the stoma aperture 64. Location of the arrangement 61 will be achieved through adhesive patches 64 secured to a user's abdomen. Normal gas and waste effusions will occur through the aperture 63 into the bag created by the layer 62, 65 and gas presented in this bag can pass through the filter vent 66 into the pocket 69. As indicated, this pocket will expand with gas with the apertures in the pocket through normal aspiration slowly releasing gas. If a user becomes aware of the bag 61 expanding significantly, conditions within the bag can be determined through an initial gentle depression in the direction of arrowhead Y. If the depression results in significant resistance this will indicate that water and solid wastes have been collected in the bag formed by the layers 62, 65. If depression receives little resistance then it will be understood that the bag and, in particular, the pocket 69 are filled with gas and further slightly more forceful depression will release that gas through the apertures 71.

As indicated above, generally the natural gas will be released from the pocket 69 through apertures 71 formed in the walls 70 as well as the top 72 but it will also be understood that the material from which the layer 68 is formed may be a textile or other fibrous material and, therefore, pores within this material may slowly release natural gas. The material may be scented. It will be appreciated that expansion of the pocket 69 will occur when the rate of gas transmission through the filter 66 exceeds the normal aspirate natural gas rate released through the pores in the material of the cover 68 as well as any normal opening or apertures.

## Claims

1. A colostomy arrangement comprising a bag arrangement (1) for a colostomy stoma, the bag arrangement comprising a bag having a stoma opening (3) and a vent filter (6), the arrangement including a hood (8) at least secured over the vent filter, the hood including an adaptor (9) to allow gaseous withdrawal through the vent filter, **characterised in that** the colostomy arrangement further comprises a gaseous withdrawal pump (48).

2. A colostomy arrangement according to claim 1, wherein the gaseous withdrawal pump (48) comprises a funnel (49) arranged in use to be secured to the adaptor (9).

3. A colostomy arrangement according to claim 1 or claim 2 wherein the gaseous withdrawal pump comprises an impeller operable in use to draw gas into the pump.

4. An arrangement as claimed in any preceding claim wherein the hood (8) substantially surrounds the whole bag arrangement with an aperture through which the stoma opening projects.

5. An arrangement as claimed in any preceding claim wherein the filter (6) is directly aligned with the adaptor (9).

6. An arrangement as claimed in any preceding claim wherein the filter is impregnated with a scenting agent.

7. An arrangement as claimed in any preceding claim wherein the filter is integrally secured to the bag.

8. An arrangement as claimed in any preceding claim wherein the filter provides a moisture barrier when wet.

9. An arrangement as claimed in any preceding claim wherein the filter and/or the adaptor are arranged to regulate rates of gaseous withdrawal in use.

## Patentansprüche

1. Kolostomie-Anordnung, welche eine Beutel-Anordnung (1) für ein Kolostomie-Stoma aufweist, wobei die Beutel-Anordnung einen Beutel mit einer Stoma-Öffnung (3) und einem Entgasungsfilter (6) aufweist, und wobei die Anordnung eine zumindest über dem Entgasungsfilter befestigte Haube (8) enthält, welche einen Adapter (9) enthält, um das Abziehen von Gas durch das Entgasungsfilter zu ermöglichen, **dadurch gekennzeichnet, dass** die Kolostomie-Anordnung ausserdem eine Gasabzug-Pumpe (48) aufweist.

2. Kolostomie-Anordnung nach Anspruch 1, **dadurch gekennzeichnet, dass** die Gasabzug-Pumpe (48) einen Trichter (49) aufweist, der während des Gebrauchs zum Befestigen an dem Adapter (9) angeordnet ist.

3. Kolostomie-Anordnung nach Anspruch 1 oder 2, **dadurch gekennzeichnet, dass** die Gasabzug-Pumpe einen Impeller aufweist, der während des Gebrauchs zum Einsaugen von Gas in die Pumpe betätigbar ist.

4. Anordnung nach einem der vorhergehenden Absprüche, **dadurch gekennzeichnet, dass** die Haube (8) im Wesentlichen die ganze Beutel-Anordnung mit einer Öffnung, durch welche die Stoma-Öffnung ragt, umgibt.

5. Anordnung nach einem der vorhergehenden Ansprüche, **dadurch gekennzeichnet, dass** das Filter (6) direkt mit dem Adapter (9) ausgerichtet ist.

6. Anordnung nach einem der vorhergehenden Ansprüche, **dadurch gekennzeichnet, dass** das Filter mit einem Geruchsstoff imprägniert bzw. getränkt ist.

7. Anordnung nach einem der vorhergehenden Ansprüche, **dadurch gekennzeichnet, dass** das Filter an dem Beutel einstückig befestigt ist.

8. Anordnung nach einem der vorhergehenden Ansprüche, **dadurch gekennzeichnet, dass** das Filter im nassen Zustand eine Feuchtigkeitsbarriere bildet.

9. Anordnung nach einem der vorhergehenden Ansprüche, **dadurch gekennzeichnet, dass** das Filter und/oder der Adapter zum Regeln bzw. Einstellen von Gasabzug-Geschwindigkeiten während des Gebrauchs angeordnet sind.

## Revendications

1. Ensemble de colostomie comprenant un aménagement de poche (1) pour une colostomie, l'aménagement de poche comprenant une poche ayant une ouverture d'abouchement (3) et un filtre de purge (6), l'ensemble comprenant un capuchon (8) au moins fixé sur le filtre de purge, le capuchon comprenant un adaptateur (9) pour permettre le retrait de gaz par le filtre de purge, **caractérisé en ce que** l'ensemble de colostomie comprend en outre une pompe de retrait de gaz (48).

2. Ensemble de colostomie selon la revendication 1, dans lequel la pompe de retrait de gaz (48) comprend un entonnoir (49) disposé en service pour être fixé à l'adaptateur (9).

3. Ensemble de colostomie selon la revendication 1 ou la revendication 2, dans lequel la pompe de retrait de gaz comprend un ventilateur actionnable en service pour aspirer du gaz dans la pompe.

4. Ensemble selon l'une quelconque des revendications précédentes, dans lequel le caouchon (8) entoure sensiblement tout l'aménagement de poche avec une ouverture travers laquelle fait saillie l'ouverture d'abouchement.

5. Ensemble selon l'une quelconque des revendications précédentes, dans lequel le filtre (6) est directement aligné sur l'adaptateur (9).

6. Ensemble selon l'une quelconque des revendications précédentes, dans lequel le filtre est imprégné d'un agent parfumant.

7. Ensemble selon l'une quelconque des revendications précédentes, dans lequel le filtre est fixé d'une seule pièce sur la poche.

8. Ensemble selon l'une quelconque des revendications précédentes, dans lequel le filtre offre une barrière anti-humidité lorsqu'il est mouillé.

9. Ensemble selon l'une quelconque des revendications précédentes, dans lequel le filtre et/ou l'adaptateur est ou sont aménagés pour réguler les débits de retrait de gaz en service.
